# EUROPEAN PATENT APPLICATION

(11) **EP 1 407 751 A1**
(43) Date of publication of application: **14.04.2004**
(21) Application number: 02743742.5
(22) Date of filing: 27.06.2002
(51) Int. Cl.: A61K 7/00, A61K 7/48

(54) **SHEET-TYPE PACKS**

(30) Priority: 27.06.2001 JP 2001194141
(71) Applicant: HISAMITSU PHARMACEUTICAL CO. INC., Tosu-shi, Saga-ken 841-0017 (JP)
(72) Inventor: HINOTANI, Tomoyuki, Tosu-shi, Saga 841-0017 (JP); MUTA, Kazunori, Tosu-shi, Saga 841-0017 (JP)
(74) Representative: Schnappauf, Georg Dr.
(86) International application number: PCT/JP2002/006471
(87) International publication number: WO 2003/002075

(57) **Abstract**

It is intended to provide sheet-type packs which have an effect of inhibiting pigmentation, spots and freckles after sunburn as well as a skin whitening effect while moisturizing the skin and maintaining adequate skin-moistening properties. Namely, sheet -type packs wherein at least a crude drug component and N-acetyltyrosine are blended with a base.

## Description

### Technical Field

The invention relates to sheet-type packs containing a skin-care action and a skin whitening action in a base. More particularly, it relates to the sheet-type packs wherein a skin-whitening component is blended in a base comprising a water-soluble polymer, a polyhydric alcohol, a crosslinking agent, water and a skin-care component.

### Background Art

In recent years, a pack excellent in a water holding capacity having a low removal strength, which contains polyacrylates, polyhydric alcohols and water as main components, has been described in JP. A. 54-49334 as a pack preparation. Also, in JP. B. H1-46485 a sheet-type pack using a cross-linking type aqueous gel as a base material and further in JP. A. H5-295004 a pack mixed with a naturally derived semisynthetic component as a moisturizing agent and a thickner are disclosed.

However, a conventional pack has not been examined totally in aspects such as moisturizing the skin and a skin-moistening property, releasability of the moisturizing component, further a skin whitening effect and inhibition of pigmentation, spots and freckles.

### Disclosure of the Invention

To solve the above problems to date, the invention makes it an object to provide sheet-type packs which have an effect of inhibiting pigmentation, spots and freckles after sunburn as well as a skin whitening effect while moisturizing the skin and maintaining adequate skin-moistening properties.

The inventors found out that a sheet-type pack wherein a crude drug component and N-acetyltyrosine are blended have an effect of inhibiting pigmentation, thereby inhibiting the occurrence of spots and freckles due to sunburn and showing a remarkable effect in a skin whitening effect. Further, the inventors found out that this sheet-type pack, in which a skin irritation is very weak in the skin safety test, is one remarkably excellent in safety, and have thus accomplished the invention.

Namely, the invention relates to a sheet-type pack, wherein at least a crude drug component and N-acetyltyrosine are blended with a base.

Also, the invention relates to the above sheet-type pack, the base comprising a water-soluble polymer, a polyhydric alcohol, a crosslinking agent, water and a skin-care component.

Further, the invention relates to the above sheet-type pack, wherein the water-soluble polymer is gelatin and/or partial neutralization products of polyacrylic acid.

Also, the invention relates to the above sheet-type pack, wherein the polyhydric alcohol is polyethylene glycol and/or polypropylene glycol.

Further, the invention relates to the above sheet-type pack, wherein the crosslinking agent is a slightly water-soluble aluminum compound and/or a polyfunctional epoxy compound.

Also, the invention relates to the above sheet-type pack, comprising as the skin care component one or more species selected from the group consisting of water-soluble placenta extract, allantoin, horse chestnut extract, kojic acid, lecithin, amino acids, placenta extract, vitamins, hormone preparations, antiallergic agents and anti-inflammatory agents.

Further, the invention relates to the above sheet-type pack, comprising as the crude drug component one or more species selected from the group consisting of moutan bark, pueraria, Asiatic ginseng, ginkgo, peony, Japanese angelica root, cnidum rhizome, clove, Swertia herb, atractylodes lancea rhizome, citrus unshu peel, capsicum, atractyrodes rhizome, orange peel, cinnamon, goldthread, phellodendron bark, schizonepeta spike and *Simomenium acutum*.

Also, the invention relates to the above sheet-type pack, comprising moutan bark and pueraria as the crude drug component.

Further, the invention relates to the above sheet-type pack, wherein the blended amount of the crude drug component is 0.00001-1.1 % by mass.

Also, the invention relates to the above sheet-type pack, wherein the blended amount of N-acetyltyrosine is 0.0002-0.1 % by mass.

Further, the invention relates to the above sheet-type pack, wherein the total blended amount of the crude drug component and N-acetyltyrosine is 0.0005-0.1 % by mass.

Also, the invention relates to the above sheet-type pack, which is a sheet-type pack used for skin whitening.

### Mode for carrying out the Invention

The sheet-type pack of the invention is one, wherein at least a crude drug component and N-acetyltyrosine are blended with a base.

Although the base which can be used for the sheet-type pack of the invention is not particularly limited, it is preferably one comprising a water-soluble polymer, a polyhydric alcohol, a crosslinking agent, water and a skin-care component.

Illustrative of the water-soluble polymers are gelatin, polyacrylic acid or salts thereof, partial neutralization products thereof or the like, and each can be used individually or by blending more than two species. As the polyacrylic acid salts, salts of metals such as sodium, lithium and potassium are preferable, and one whose average degree of polymerization is 1000-100000 is expediently used. Among these water-soluble polymer bases, a blend by combination of gelatin and partial neutralization products of polyacrylic acid is particularly preferable.

As a blended amount of these water-soluble polymers, it is used in 1-25 % by mass, preferably 3-25 % by mass, and more preferably 5-20 % by mass, in particular preferably 5-10 % by mass from the viewpoints of such as adhesiveness, agglutinativeness of the preparation, a shape retaining property and water absorption power, and further from viewpoints such as viscosity during manufacturing, non-uniformity of paste, improvement of workability and usability.

The polyhydric alcohol consists of blend of glycols, and the blended amount based on the total amount of the base is 1-35 % by mass, preferably 5-25 % by mass, and more preferably 5-20 % by mass from the viewpoints of such as an adhesiveness and an agglutinativeness of a preparation, a water holding and shape retaining properties before use, uniformity of gel, workability and usability. Further, as the glycols in the polyhydric alcohol, polyethylene glycol of the average molecular weight 200-600, polypropylene glycol of the average molecular weight 500-3000 and the like having an polyether structure are preferable, and one or more species of these can be used by blending.

As the crosslinking agent, one or more species of a slightly water-soluble aluminum compound or a polyfunctional epoxy compound can be used by blending. Illustrative of the slightly water-soluble aluminum compounds are aluminum hydroxide, aluminum silicate hydrate, synthetic aluminum silicate, kaolin, aluminum acetate, aluminum lactate, aluminum stearate, dry aluminum hydroxide gel, magnesium metasilicate aluminate, magnesium silicate aluminate, aluminum dihydroxy aminoacetate, synthetic hydrothalsulfite, aluminum magnesium silicate and the like, and one or more species of these can be used by blending. Use of the slightly water-soluble aluminum compounds gives gel an appropriate strength in an initial physical property as a filler in addition to an inhibitory effect for skin irritancy by the antacid action and a skin astringent action by trace aluminum ion, and along with this, aluminum ion dissolves into the preparation in a time course, thereby it is possible to show a function to cover the lowering of the gel strength owing to time dependent decomposition of polymer and time dependent cleavage of a cross-linking part of covalent bondings between polymers. Further, the aluminum dissolution rate can be controlled by adjusting pH.

Illustrative of the polyfunctional epoxy compounds are polyethyleneglycol diglycidyl ether, ethyleneglycol diglycidyl ether, glycerin diglycidyl ether, glycerin triglycidyl ether, propyleneglycol diglycidyl ether, polyglycerol polyglycidyl ether, sorbitol polyglycidyl ether, sorbitan polyglycidyl ether, trimethylolpropane polyglycidyl ether, pentaerythritol polyglycidyl ether, resorcinol diglycidyl ether, neopentylglycol diglycidyl ether and the like. One or more species of these polyfunctional epoxy compounds can be used by blending. An excellent water absorption and shape retaining property can be obtained by use of the polyfunctional epoxy compounds, and they can produce covalent bonding with the water soluble polymers having a carboxyl, amino or hydroxyl groups, or the like, enhancing a gel strength.

As the blended amount of these crosslinking agents, that is, the slightly water-soluble aluminum compounds and/or the polyfunctional epoxy compounds, it is used in 0.01-20 % by mass, preferably 0.02-15 % by mass, and more preferably 0.05-10 % by mass from the viewpoints of such as agglutinativeness and a shape retaining property of the preparation, water absorption power, time dependent stability in a physical property of the reparation, workability, safety for the skin and usability. Further, considering physical properties, real feeling of use or an effect of use in the preparation, use of the slightly water-soluble aluminum compound together with the polyfunctional epoxy compound is preferable.

As water, purified water, sterile water or natural water is used. Water acts as a dispersion-dissolution agent for a skin-care component, a moisturizing component, a water-soluble polymer, a cross-linking agent, an antiseptic and the like, and is especially important to disperse and dissolve the skin-care component and the moisturizing agent uniformly in the preparation. Further, water itself increases usability during use and after use, and moves into the skin together with the moisturizing agent, bringing an effect to give moisture and tension. From these viewpoints and viewpoints of such as adhesiveness, agglutinativeness, a water holding property, a shape retaining property, workability and usability of the preparation, the blended amount of water is 50-95 % by mass, preferably 60-95 % by mass, more preferably 65-90 % by mass, and in particular preferably 70-85 % by mass, to be added in a large amount. The relative humidity of the preparation itself can be heightened by having it contain a large amount of water in it, and it becomes possible to drain off effectively a large amount of water into the outside, consequently giving moisture to the skin and depriving of the heat of vaporization by evaporation of water to the outside, whereby it is possible to afford comfortable refreshing feeling.

Illustrative of the skin-care components are allantoin, horse chestnut extract, water-soluble placenta extract, sage extract, kojic acid, lecithin, amino acids, proteins, saccharides, hormones, placenta extract, or extraction components from various crude drugs such as aloe, mulberry, loofah and glycyrrhia, or vitamin A, vitamin C, vitamin D, vitamin E and derivatives thereof, other vitamins or derivatives thereof, or dipotassium glycyrrhizinate, diphenhydramine hydrochloride, diphenhydramine salycilate, diphenhydramine tannate, triprolidinehydrochloride, mequitazine, chlorpheniramine maleate, d-chlorpheniramine maleate, clemastine fumarate, promethazine hydrochloride, tranilast, sodium cromoglycate, ketotifen, arylsulfatase B, bufexamac, bendazac, butyl flufenamate, ibuprofen piconol, indomethacin, aspirin, flurbiprofen, ketoprofen, piroxicam, 2-pyridinemethyl mefenamic acid, 5,6-dehydroarachidonic acid, 5,6-methano-LTA₄, esculetin, eupatilin, 4-demethyleupatilin, caffeic acid or benoxaprofen, and one or more species of these can be used by blending.

As the mix amount of the skin-care components, it is used in 0.001-10 % by mass, preferably 0.005-5 % by mass, and more preferably 0.01-1 % by mass from the viewpoints of workability, usability and use effect, and further from the viewpoints of such as adhesiveness and agglutinativeness of the preparation, a shape retaining property, non-uniformity of paste and safety.

As the crude drug components it can be selected from moutan bark (including moutan extract and moutan powder), pueraria, peony, Japanese angelica root, cnidum rhizome, clove, Swertia herb, atracyrodes lancea rhizome, citrus unshu peel, capsicum, atracyrodes rhizome, orange peel, cinnamon, goldthread, phellodendron bark, ginkgo, schizonepeta spike and *Simomenium acutum*, and one or more species can be blended. As the crude drug component, it is 0.00001-1.1 % by mass, preferably 0.0001-0.5 % by mass, and more preferably 0.001-0.1 % by mass based on the total amount of the base from the viewpoints of such as a use effect of the crude drug component, usability, smell due to the crude drug, and production costs.

Also, N-acethytyrosine is blended, the blending amount being 0.00001-1 % by mass, preferably 0.0001-0.5 % by mass, and more preferably 0.0002-0.1 % by mass from the viewpoints of such as adhesiveness, and agglutinativeness of the preparation, a shape retaining property, and production costs.

Further, the blended amount of N-acethytyrosine and one or more species of the crude drug components selected from the crude drugs such as moutan bark and pueraria described above is in the range of 0.0001-1.1 % by mass and preferably 0.0005-0.1 % by mass based on the total amount of the base. A three component system of moutan bark, pueraria and N-acethytyrosine is particularly preferable, and the three components are preferably blended in the ratio of moutan bark : pueraria : N-acethytyrosine = 25-45 : 10-20 : 30-60. By blending the mixture liquid of mixed plant extraction liquid-amino acids (the trade name, manufactured by Ichimaru pharcos Co., Ltd.; in this product a purified water and 1,3-buthylene glycol is 98.88 % by mass as the base component, and the remaining 1.12 % by mass is the content of the three component system.), which mixed plant extraction consisting of said three component system in 0.01-5 % by mass, and preferably 0.01-1 % by mass, in particular spots and freckles are remarkably inhibited by an inhibiting action of pigmentation. Also, it has a remarkable effect in skin whitening.

Further, in addition to the above base components the sheet-type pack of the invention can appropriately be blended in a suitable amount with an antiseptic, moisturizing component, antioxidant, adhesiveness donating agent, dissolution agent, pigment, perfume, surfactant, UV absorber, an inorganic filler, pH adjusting agent and the like known in the art.

Illustrative of the antiseptics are p-hydroxybenzoic acid ester (for example, methylparaben, ethylparaben, propylparaben), 1,2-pentanediol, benzoic acid, benzoate, salicylate, sorbic acid, sorbate, dehydroacetate, 4-isopropyl-3-methylphenol, 2-isopropyl-5-methylphenol, phenol, hinokitiol, cresol, 2,4,4'-trichloro-2'-hydroxydiphenyl ether, carbanide, 3,4,4'-trichlorocarbanide, chlorobutnol, benzalkonium chloride, benzethonium chloride, etc., and one or more species of these can be used by blending. p-Hydroxybenzoic acid ester is preferable among these.

As the blended amount of the antiseptics, it is used in 0.005-10 % by mass, preferably 0.01-5 % by mass, and more preferably 0.01-1 % by mass from the viewpoints of such as putrefaction of the preparation during storage and usability when using and after using, and further from the viewpoints of such as adhesiveness and agglutinativeness, skin irritancy and safety in the preparation.

As the moisturizing can be blended one or more species of rose fruit extract, orange extract, orange juice, raspberry extract, kiwi extract, cucumber extract, gardenia extract, grape fruit extract, crataegus fruit extract, crataegus extract, common juniper extract, duke extract, tomato extract, grape extract, loofah extract, lime juice, apple extract, apple juice, lemon extract, lemon juice, carrot extract, aqueous succinyl kefiran solution, aqueous acetyl kefiran solution, aqueous maleyl kefiran solution, malt root extract, rose extract, collagen, ceramide, hyaluronic acid, etc. In particular, as to fruit extracts (fruit juices) they have effects as a perfume, and have action to heighten the effect of the refreshing agent and the perfume which are essential components.

As the antioxidants can be blended sodium edetate, ascorbic acid, propyl gallate, butylhydroxyanisol, dibutyl hydroxy toluene, nordihydroguaretic acid, tocopherol, tocopherol acetate and the like.

As the tackifiers can be blended casein, pullulan, agar, dextran, sodium alginate, soluble starch, carboxystarch, dextrin, carboxymethyl cellulose, sodium carboxymethyl cellulose, methylcellulose, ethylcellulose, hydroxyethylcellulose, polyvinyl alcohol, polyethylene oxide, polyacrylamide, polyacrylic acid, polyvinylpyrrolidone, carboxyvinyl polymer, polyvinyl ether, polymaleic acid copolymer, methoxyethylene maleicanhydride copolymer, isobutylene maleicanhydride copolymer, polyethylene imine and the like.

As the dissolution agents can be blended benzyl alcohol, pyrrothiodecane, peppermint oil, isopropyl myristate, crotamitone and the like.

As the pigments can be blended the officially designated pigments such as Red No.2 (amarnath), Red No.3 (erythrosine), Red No.102 (new coccine), Red No.104-1 (phloxine B), Red No.105-1 (rose bengale), Red No.106 (acid red), Yellow No.4 (tartrazine), Yellow No.5 (sunset yellow FCF), Green No.3 (fast green FCF), Blue No.1 (brilliant blue FCF), Blue No.2 (indigo carmine) and the like. As for the pigments they are not particularly limited, though they give a great influence to the preparation image and result to the improvement of usability and activation feeling to the skin.

As the surfactants can be blended anionic surfactants such as sodium dioctyl sulfosuccinate, alkylsulfate salts, 2-ethylhexyl alkylsulfate ester sodium salt or sodiumu n-dodecyl benzenesulfonate, cationic surfactants such as hexadecyl trimethylammonium chloride, octadecyl dimethyl benzyl ammonium chloride or polyoxyethylene dodecyl monomethylammonium chloride, and nonionic surfactants such as polyoxyethylene stearylether, polyoxyethylene tridecylether, polyoxyethylene nonylphenylether, polyoxyethylene octylphenylether, polyoxyethylene monostearate, sorbitan monostearate, sorbitan monopalmitate, sorbitan sesquioleate, polyoxyethylene sorbitan monolaurate, polyoxyethylene sorbitan monooleate, glycerol monostearate, polyglycerin fatty acid esters or polyoxyethylene octadecyl amine.

As the UV absorbers can be blended p-aminobenzoic acid, p-aminobenzoate, amyl p-dimethylaminobenzoate, salicylate, methyl anthranilate, umbelliferone, esculin, benzyl cinnamate, cinoxate, guaiazulene, urocanic acid, 2-(2-hydroxy-5-methylphenyl)benzotriazole, 4-methoxybenzophenone, 2-hydroxy-4-methoxy benzophenone, dioxybenzone, octabenzone, dihydroxy dimethoxybenzophenone, slisobenzone, benzoresorcinol, octyl p-dimetylaminobenzoate, ethylhexyl p-methoxycinnamate, and the like.

As the inorganic fillers can be blended titanium oxide, talc, zinc oxide, silicate hydrate, magnesium carbonate, calcium hydrogen phosphate, magnesium silicate, diatomaceous earth, anhydrous silicic acid, bentonite and the like.

As the pH adjusting agent can be blended acetic acid, formic acid, lactic acid, tartaric acid, oxalic acid, benzoic acid, glycolic acid, malic acid, succinic acid, hydrochloric acid, nitric acid, sulfuric acid, sodium hydroxide, potassium hydroxide, methylamine, ethylamine, propylamine, dimethylamine, diethylamine, dipropylamine, trimethylamine, triethylamine, tripropylamine, monomethanolamine, monoethanolamine, monopropanolamine, dimethanolamine, diethanolamine, diprpopanolamine, trimethanolamine, triethanolamine, tripropanolamine and the like.

The pH of an paste appropriately blended with each of the above components in a suitable amount is desired to consider not giving irritancy to the skin, and the pH when diluting the paste 5g with a purified water to 100 ml is in the range of 5-8, preferably 5.5-7.5, and more preferably 6-7.

Further, illustrative of the backing on which the paste is applied is a flexuous one such as a synthetic resin film of polyethylene, polypropylene, polyethylene terephthalate, ethylene-vinylacetate copolymer, vinyl chloride, polyurethane, polyester, polyamide, rayon, polyester or the like, elastic nonwoven fabric, nonwoven paper, laminate of nonwoven fabric or nonwoven paper with the above synthetic resin film or sheet, nonwoven fabric such as absorbent cotton or the like, cloth, elastic cloth, paper, cellophane or the like, and it can appropriately be selected according to the way of application. Further, a pack agent layer is applied on the base fabric consisting of a flexuous backing and the surface of this pack agent layer is covered further with a removable film or paper, whereby the stability of the preparation can be kept. Also, as to the removable paper, in order to render easy adhesion to the face a separation line, perforating or the like are set, making the form easy for separation and application. Furthermore, color of the base fabric is not particularly limited, though it greatly affects image of the preparation and improves usability and activation feeling to the skin in a large degree, and white, skin color, yellow, red, orange, green, blue, pink, light blue, brown or the like can be cited, and if necessary a shade is preferably adjusted.

As the process for preparing the sheet-type pack of the invention, the above components are uniformly mixed and/or dissolved in a stirring machine and spread on the non-dyed or dyed base fabric, thereon a removable paper is stuck, and it is cut in a shape of the face. Further, parts for the eyes, nose, mouth and tin are cut in an appropriate shape, whereby it is processed for the convenience for handling. Also, aiming to use it in a part of the face it can be processed into a shape to be applied well to the aimed part as a nose pack which is applied to the nose or as an eye-around pack which is applied around the eyes. Further, a sheet-type pack is desirably preserved in a sealed bag or container until use from the view point that contamination under preservation, decrease of effectiveness by the evaporation of the volatile substance, or the like is prevented.

### Example

In the following, the sheet-type packs of the invention are explained in more detail by the examples and the test examples. However, the invention is not limited in any way by these.

### Example 1

Synthetic aluminum silicate 5 % by mass is dispersed in purified water 71.33 % by mass. This is added with gelatin 1 % by mass, ethyleneglycol diyglycidyl ether 0.07 % by mass, the mixture liquid of mixed plant extraction liquid and amino acids [trade name, manufactured by Ichimaru pharcos Co., Ltd.; in this product purified water and 1,3-butylene glycol is contained in 98.88 % by mass as the base component, and the remaining 1.12 % by mass is the three components (moutan extract, pueraria extract and N-acethytyrosine)] 0.5 % by mass, water-soluble placenta extract 1 % by mass, allantoin 0.1 % by mass and methylparaben 0.5 % by mass, which are dissolved, further being added with a mixture of partial neutralization products of polyacrylic acid 6 % by mass and polyethylene glycol 12 % by mass, and stirred till homogeneity. Subsequently, the mixture was spread on a base fabric dyed in a pale yellow color with the depth of about 1.4 mm and was stuck with a film. Further, after sticking, it was cut into a shape of the face, and parts of the eyes, nose, mouth and tin are cut into an appropriate shape to provide the sheet-type pack.

**Table 1**

| Example Components (%) | 1 |
|---|---|
| Mixture liquid of mixed plant extraction liquid and amino acids | 3 |
| Water-soluble placenta extract | 1 |
| Allantoin | 0.1 |
| Polyethylene glycol | 12 |
| Gelatin | 1 |
| Partial neutralization products of polyacrylic acid | 6 |
| Synthetic aluminum silicate | 5 |
| Ethyleneglycol diglycidyl ether | 0.07 |
| Methylparaben | 0.5 |
| Water | 71.33 |

### Example 2-9

The sheet-type packs were obtained by preparation in the same way as Example 1 using the blend agents and the blended amounts shown in Tables 2 and 3. The component (%) means % by mass.

**Table 2**

| Examples Components(%) | 2 | 3 | 4 | 5 | 6 |
|---|---|---|---|---|---|
| Mixture liquid of mixed plant extraction liquid and amino acids | 3 | | | | 1 |
| N-Acetyltyrosine | | 0.03 | 0.1 | 0.1 | |
| Moutan bark | | 0.02 | | 0.05 | 1 |
| Pueraria | | 0.01 | | | |
| Peony | | | | 0.05 | |
| Clove | 0.1 | | | | |
| Orange peel | | | | 0.1 | |
| Water-soluble placenta extract | | | | 0.09 | 0.25 |
| Allantoin | 0.05 | 0.1 | 0.04 | | 0.5 |
| Polyethylene glycol | 5 | 5 | | 20 | |
| Polypropylene glycol | 5 | | 10 | 15 | 12 |
| Gelatin | 1 | 1 | 1.5 | 5 | 0.5 |
| Polyacrylic acid | | 2 | | | |
| Polyvinyl alcohol | | 3 | | | |
| Partial neutralization products of polyacrylic acid | 10 | 5 | 10 | 5 | 8 |
| Kaolin | | 2 | 8 | | |
| Aluminum acetate | | | | 0.05 | |
| Synthetic aluminum silicate | 6 | 6 | 10 | | 4 |
| Magnesium metasilicate aluminate | | | | | 1 |
| Polyethyleneglycol diglycidyl ether | | 0.25 | | | |
| Propyleneglycol diglycidyl ether | 0.04 | | | | |
| Glycerin diglycidyl ether | | 0.025 | | | |
| Polyglycerol polyglycidyl ether | | | | 0.05 | |
| Glycerin triglycidyl ether | | | 0.05 | | |
| Methylparaben | 0.3 | 0.1 | 0.01 | | |
| Propylparaben | | 0.1 | | 0.1 | 0.5 |
| Sodium edetate | | | | | 0.5 |
| Citric acid | | | | | 0.2 |
| Perfume | 0.001 | | | | |
| Pigment | | 0.001 | | | |
| Water | 68.509 | 78.589 | 60.3 | 54.41 | 70.55 |

**Table 3**

| Examples Components(%) | 7 | 8 | 9 | 10 | 11 |
|---|---|---|---|---|---|
| Mixture liquid of mixed plant extraction liquid and amino acids | | | | 0.05 | 5 |
| N-Acetyltyrosine | 0.02 | | | | |
| Moutan bark | 0.01 | 0.03 | 0.03 | | |
| Pueraria | 0.1 | 0.02 | 0.02 | | |
| Japanese angelica root | | 0.01 | 0.01 | | |
| Peony | | | | 0.02 | |
| Clove | | | | 0.01 | |
| Orange peel | | | | 0.04 | |
| Water-soluble placenta extract | | 0.01 | 0.01 | | |
| Cinnamon | 1 | | | 1 | 1 |
| Allantoin | 0.05 | 0.1 | 0.04 | 0.1 | 0.1 |
| Polyethylene glycol | 15 | 5 | 5 | 5 | |
| Polypropylene glycol | | 5 | 5 | | 10 |
| Gelatin | 2 | 2 | 3 | 2 | 1 |
| Polyacrylic acid | | 2 | 10 | | |
| Polyvinyl alcohol | | 4 | | | |
| Partial neutralization products of polyacrylic acid | 6 | | 10 | | 5 |
| Kaolin | | 4 | | 4 | |
| Aluminum acetate | | | | | 0.05 |
| Synthetic aluminum silicate | 3 | 2 | | 2 | 4 |
| Magnesium metasilicate aluminate | 0.5 | | | | |
| Sorbitol polyglycidyl ether | | | 0.05 | | |
| Polyethyleneglycol diglycidyl ether | | 0.025 | | | |
| Propyleneglycol diglycidyl ether | 0.02 | | | 0.1 | 0.025 |
| Glycerin diglycidyl ether | | 0.025 | | | |
| Polyglycerol polyglycidyl ether | | | | | 0.05 |
| Glycerin triglycidyl ether | | | | | |
| Methylparaben | 0.3 | 0.1 | | 0.05 | 0.05 |
| Propylparaben | | 0.1 | 0.01 | | |
| Sodium edetate | 0.4 | | | | |
| Citric acid | 0.2 | | | | |
| Perfume | 0.001 | | | | |
| Pigment | | 0.001 | | | |
| Water | 71.399 | 75.579 | 66.83 | 85.63 | 73.725 |

### Comparative Example 1 Without the mixture liquid of mixed plant extraction liquid and amino acid (trade name)

A sheet-type pack was prepared in the same formulation as that of Example 1 except that the mixture liquid of mixed plant extraction liquid and amino acids (trade name) was excluded from the formulation of Example 1 and the blended amount of purified water was made 74.33 % by mass.

### Comparative Example 2

A sheet-type pack was prepared in the same formulation as that of Example 1 except that the blended amount of polyethylene glycol was changed from 12 % by mass to 6 % by mass, the blended amount of gelatin from 12 % by mass to 6 % by mass, that of partial neutralization products of polyacrylic acid from 6 % by mass to 3 % by mass, and aluminum silicate from 5 % by mass to 2.5 % by mass.

### Test Example 1 Adhesiveness test

As for Examples 1,2,8 and 9, the results of the adhesiveness test carried out are shown in Table 4. In the test used were samples which were allowed to stand previously for not less than 30 min under the condition of 25°C-60%Rh, and the test was carried out under the same conditions. First, the sample was fixed on a horizontal stand with the adhesive surface exposed to the outside. Subsequently, a steel ball whose diameter was 20/32 inch was dropped along the sine curve from the height of 17.35 cm for the length of 30 cm on the base and was rolled on the adhesive surface. The distance (cm) from the ground point of the steel ball to the reaching point was employed as the parameter to evaluate the stick strength. Meanwhile, with respect to the steel ball used, the material thereof was SUJ2 of JIS G4805(high carbon chromium bearing steel) and the precision was the upper class of JIS B1501 (steel ball for ball bearing).

**Table 4**

| | Adhesive strength |
|---|---|
| Example 1 | 3.5 cm |
| Example 2 | 3.0cm |
| Example 8 | 5.0 cm |
| Example 9 | 6.5 cm |

### Test example 2 Evaluation of skin whitening effect

As for Example 1 and Comparative Example 1, the skin whitening effect test was carried out. The test was carried out with twenty women in the age of twenties to forties having spots for Example 1 or Comparative Example 1 each, to whom either of the samples was adhered for 4 months, three times every week, thereafter the skin whitening effect was evaluated in 5 degrees.

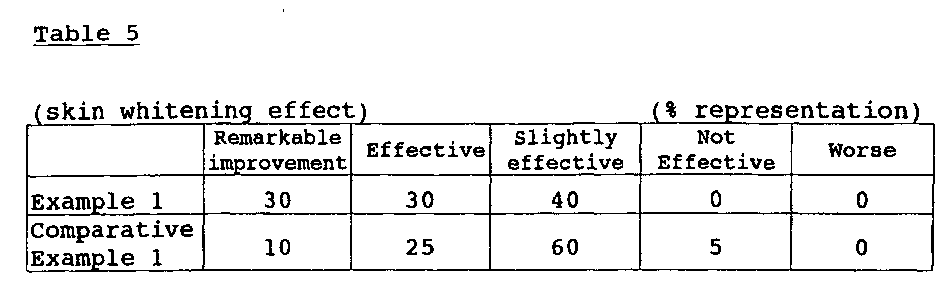

### Test Example 3 Usability evaluation test

As for Example 1 and Comparative Example 2 a usability test was carried out. In the test, 50 women in the age of twenties were provided with one sheet of both samples of Example 1 and Comparative Example 2 for each person and made to use each sheet on a different day. Then, the subjects were made to evaluate in 5 degrees on the following items: smooth application feeling over the skin, efficacy and penetration feeling, and relaxed feeling. The test results are shown in Table 6 (smooth application feeling over the skin), in Table 7 (efficacy and penetration feeling) and in Table 8 (relaxed feeling).

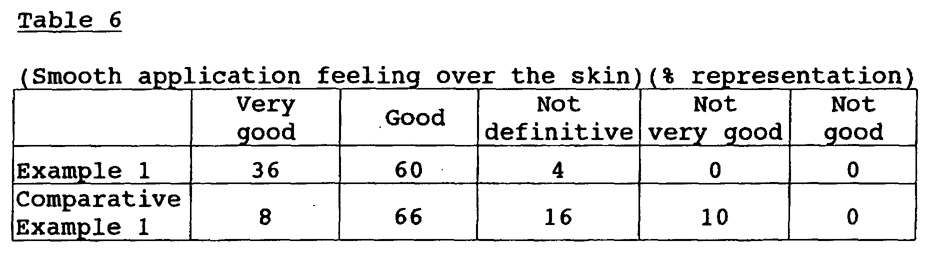

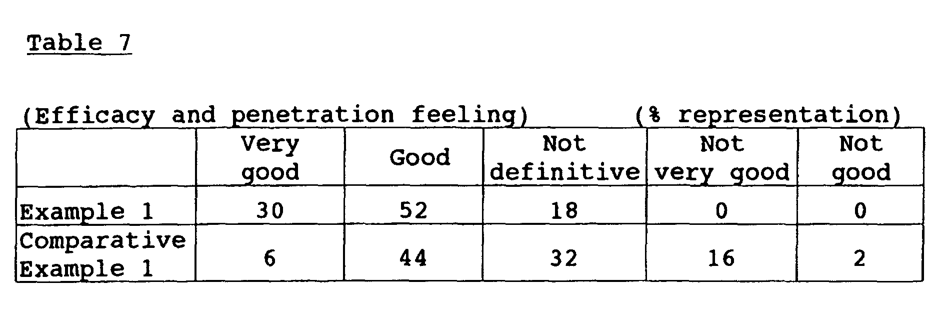

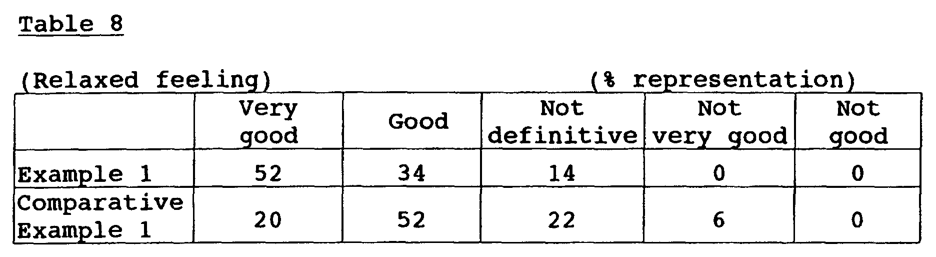

### Test Example 4 Skin safety test

As for Examples 1, 2 and 3 and the comparative example 1 the skin safety test was carried out. A 48-hr closed patch test was carried out with 30 healthy men and women. Changes of the skin were observed after 1 hr. and 24 hrs. after the detachment, and the irritancy degree of the skin was evaluated according to the standards below. The results are shown in Table 9.
-: No change is observed in the skin
±: Faint flare in the skin
+: Clear flare in the skin
++: Severe feeling in the skin

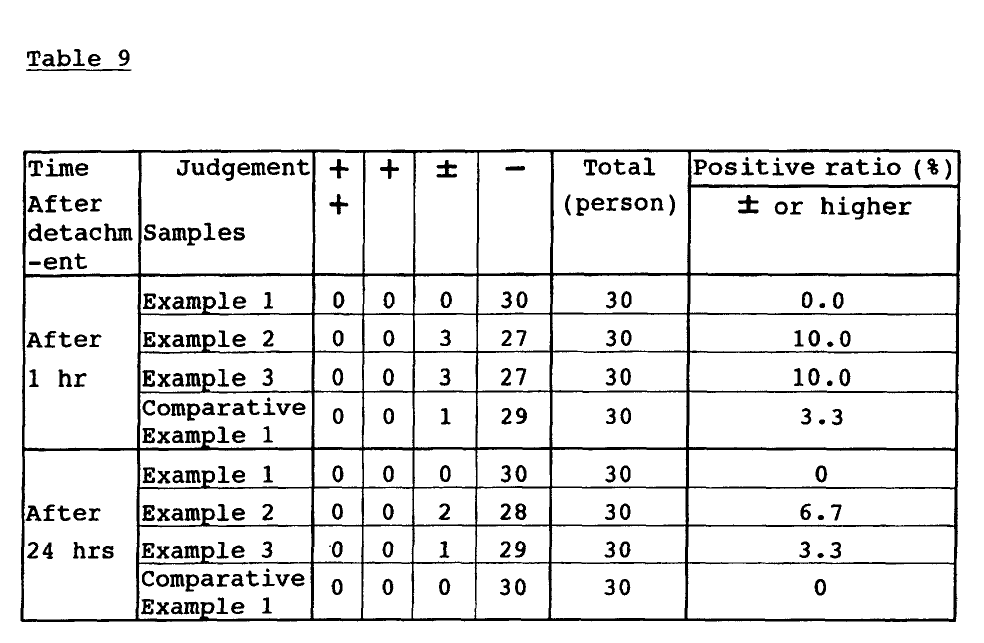

As described above, it has been proved that the sheet-type pack of the invention has adequate adhesiveness and good usability and is excellent in the skin safety. It was also proved that it is excellent in the skin-moistening and skin whitening effects.

### Industrial Applicability

The sheet-type pack of the invention has effects such as
a. Easy handling.
b. Adequate adhesiveness to the skin.
c. Excellent physical stability of the preparation.
d. Excellent safety for the skin.
e. Effect of removing skin wastes, making the skin clean.
f. Cooling effect due to its high water content, providing comfortable refreshing feeling.
g. Endothermic effect due to its high water content, and inhibitory action for glow and inflammation of the skin.
h. Excellent moisturizing action after use.
i. Skin smoothing effect.
j. Excellent in skindressing and a beautification.
k. Relaxing effect.
l. Removal of an excess oiliness and giving an adequate moisturizing. Namely, the sheet-type pack of the invention is easy to handle as well as has adequate adhesiveness to the skin, while it is excellent in safety for the skin, usability, and effect for the skin.

Further, owing to these effects, it is used for the face (entire face, in local parts such as forehead, nose, corner of eye and chin) owing to giving skin whitening, inhibition of spots and freckles by pigmentation as well as sunburn, and simultaneously moist feeling and moisture to the skin, and therefore, it can be applied in the fields of quasi-drug or cosmetics used for skindressing and beautification, and is industrially very useful.

## Claims

1. A sheet-type pack, wherein at least a crude drug component and N-acetyltyrosine are blended with a base.

2. The sheet-type pack according to claim 1, wherein the base comprises a water-soluble polymer, a polyhydric alcohol, a crosslinking agent, water and a skin-care component.

3. The sheet-type pack according to claim 2, wherein the water-soluble polymer is gelatin and/or partial neutralization products of polyacrylic acid.

4. The sheet-type pack according to claims 2 or 3, wherein the polyhydric alcohol is polyethylene glycol and/or polypropylene glycol.

5. The sheet-type pack according to any one of claims 2 to 4, wherein the crosslinking agent is a slightly water-soluble aluminum compound and/or a polyfunctional epoxy compound.

6. The sheet-type pack according to any one of claims 2 to 5, comprising as the skin-care component one or more species selected from the group consisting of water-soluble placenta extract, allantoin, horse chestnut extract, kojic acid, lecithin, amino acids, placenta extract, vitamins, hormone preparations, antiallergic agents and anti-inflammatory agents.

7. The sheet-type pack according to any one of claims 1 to 6, comprising as the crude drug component one or more species selected from the group consisting of moutan bark, pueraria, Asiatic ginseng, ginkgo, peony, Japanese angelica root, cnidum rhizome, clove, Swertia herb, atractylodes lancera rhizome, citrus unshu peel, capsicum, atracyrodes rhizome, orange peel, cinnamon, goldthread, phellodendron bark, schizonepeta spike and *Simomenium acutum*.

8. The sheet-type pack according to claim 7, comprising moutan bark and pueraria as the crude drug component.

9. The sheet-type pack according to claim 7, wherein the blended amount of the crude drug component is 0.00001-1.1 % by mass.

10. The sheet-type pack according to claims 8 or 9, wherein the blended amount of N-acetyltyrosine is 0.0002-0.1 % by mass.

11. The sheet-type pack according to claim 10, wherein the total blended amount of the crude drug component and N-acetyltyrosine is 0.0005-0.1 % by mass.

12. The sheet-type pack according to claims 1 to 11, which is a sheet-type pack used for skin whitening.
